# EUROPEAN PATENT APPLICATION

(11) **EP 4 660 165 A1**
(43) Date of publication of application: **10.12.2025**
(21) Application number: 24180147.1
(22) Date of filing: 05.06.2024
(51) Int. Cl.: C03C 4/00, A61C 5/77, C03C 10/00, F27B 17/02

(54) **PROCESS FOR PREPARING A DENTAL GLASS CERAMIC RESTORATION**

(71) Applicant: DeguDent GmbH, 63457 Hanau (DE); Dentsply Sirona Inc., York, PA 17401 (US)
(72) Inventor: Fecher, Stefan, 63457 Hanau (DE); Völkl, Lothar, 63457 Hanau (DE); Meegdes, Marcel, 63457 Hanau (DE)
(74) Representative: Crow, Martin

(57) **Abstract**

The present invention provides a process for preparing a dental restoration comprising the following steps:
(a1) providing a dental glass ceramics mill blank and machining an indirect dental restoration from the mill blank, or
(a2) providing a dental glass ceramics press blank and pressing an indirect dental restoration from the press blank; and
(b) heat treating the indirect dental restoration obtained in step (a1) or (a2) at a temperature below the melting point of the dental glass ceramics for at least 15 seconds by irradiating light having a wavelength in the range of 350 to 500 nm, for preparing the dental restoration.

Moreover, the present invention provides a dental furnace, in particular for use in the process of the present invention.

## Description

### Field of the Invention

The present invention relates to a process for preparing a dental restoration, particularly an indirect dental restoration, from a dental glass ceramics mill blank or a dental glass ceramics press blank. Moreover, the present invention relates to a dental furnace for preparing a dental restoration. Finally, the present invention relates to a use of the dental furnace of the present invention for heat treating a dental restoration comprising glass ceramics, notably in a process of the present invention.

According to the present invention, an indirect dental restoration may be prepared chairside by heat treating a milled or pressed dental glass ceramics restoration in a short time of 10 minutes or less whereby a high level of mechanical and esthetical properties is achieved with significantly less energy consumption compared to a process using a conventional dental furnace. According to a preferred embodiment, a dental glass ceramics restoration may be prepared chairside in a single appointment thereby avoiding the need for any temporary restoration.

### Background of the Invention

The preparation of glass ceramics dental restorations may use a dental glass ceramics mill blank, or a dental glass ceramics press blank.

A dental glass ceramics mill blank is a block of ceramic material typically comprising materials like lithium disilicate or leucite, known for their aesthetic qualities and mechanical strength. The mill blank is placed in a dental milling machine which uses computer-aided design (CAD) and computer-aided manufacturing (CAM) technology to mill the dental restoration from the blank. Once the milling process is completed, the milled restoration undergoes various treatments to enhance physical properties and aesthetics including heat treatment processes for facilitating crystallization to improve the strength and durability of the material. Any crystallization process must be carefully controlled, often requiring the restoration to be heated to specific temperatures that transform the glassy matrix into a more crystalline structure. Finally, the restoration may be glazed to achieve the desired surface finish before it is fitted into the patient's mouth.

Alternatively, a dental restoration may be prepared by using a dental press blank, which is often composed of lithium disilicate due to excellent optical properties and strength. The blanks are preformed into blocks or ingots that fit into a press furnace. For preparing the dental restoration, the dentist takes an impression of the patient's teeth, which is used to prepare a precise model of the teeth requiring the restoration. A wax model of the restoration is then prepared on the model of the teeth. The shape of the wax model corresponds to the shape of the final ceramic piece. The wax model is then encased in a refractory material to form a mold. Once the investment material sets, the wax is melted away, leaving a cavity in the shape of the restoration. The glass ceramic press blank is heated in a pressing furnace until it reaches a viscous state. Using a press, the molten glass ceramic is forced (pressed) into the cavity of the investment mold where the wax pattern was previously removed. The material flows and fills up every detail of the mold cavity. After pressing, the material is allowed to cool slowly within the mold, solidifying into the shape of the restoration. Once cooled, the restoration is divested and any extra material from the pressing process, known as sprues, are removed, and the restoration is cleaned. The restoration may require further adjustments for fit and occlusion and may also be polished or glazed to achieve the desired aesthetic finish. Subsequently, the restoration may undergo additional heat treatment to enhance strength and aesthetics, such as controlled crystallization processes to improve toughness and translucency.

Commonly, a patient might expect to visit the dentist multiple times for the entire process of getting a glass ceramics dental restoration. Specifically, after the dentist assesses the patient's need for a glass ceramic restoration, the affected tooth is reshaped to ensure a proper fit of the dental restoration. Accordingly, the dentist prepares the tooth, which might involve trimming or filing down the tooth. Subsequently, a digital scanning equipment will be used to create a detailed 3D model of the teeth. This model is used to design the restoration so that it fits with the patient's existing dental structure. A temporary restoration is then required to protect the tooth while the permanent restoration is being fabricated in a dental laboratory.

Based on the final design of the restoration, the restoration is shaped either through milling from a ceramic blank or by pressing in a mold. At this stage, the material is typically in a partially crystallized or amorphous (glassy) state, which allows for easier machining or molding. Subsequently, the restoration undergoes any necessary treatments such as glazing.

After the restoration is available at the dentist's office, the patient returns for a fitting. During this visit, the dentist checks the fit, color, and occlusion of the restoration, making any minor adjustments if needed. During the final visit, the restoration is permanently affixed to the patient's tooth and the dentist ensures that the restoration fits seamlessly with the adjacent teeth and that the patient's occlusion is correct.

The primary reason for heat treating glass ceramic materials is to control the crystallization process within the ceramic and to improve the surface properties by eliminating microcracks. This process is crucial for improving the strength and toughness of the final restoration, as well as achieving desired optical properties such as translucency and color matching to the patient's natural teeth.

Conventionally, specific temperatures and times for different phases of the heat treatment must be closely controlled to achieve the desired properties. For example, lithium disilicate ceramics might be heated to around 850°C for crystallization, while different ceramics might require different temperatures and times based on their unique composition. In any case the heat treatment typically requires 15 to 30 minutes and is followed by a controlled cooling to prevent thermal shock and ensure the stability of the crystalline phases formed during heat treatment. After heat treatment, additional steps such as glazing or staining may be applied to enhance the visual match to natural tooth enamel and to further improve the surface properties.

Given the time-consuming method steps in the preparation of a dental glass ceramic restoration, the number of visits of the patient at the dentist's office and the important contribution of a dental laboratory cannot be reduced and prevent any increase of the efficiency in the treatment with an indirect dental restoration.

### Summary of the Invention

According to a first aspect, it is the problem of the present invention to provide a process for preparing a dental restoration from a dental glass ceramics mill blank in a short time of less than 10 minutes while achieving a high level of mechanical and esthetical properties, so that the process may be carried out chairside by the dental practitioner in the presence of the patient whereby a temporary restoration may be avoided, and at least two visits of the patient at the dentist's office may be combined into a single appointment. A further problem of the present invention is to provide a process for preparing a dental restoration from a dental glass ceramics press blank whereby surface microdefects may be healed by a heat treatment in a short time of less than 10 minutes while achieving and maintaining a high level of mechanical and esthetical properties, so that the heat treatment may even be carried out chairside by the dental practitioner in the presence of the patient.

Moreover, it is a problem of the present invention to provide a process which requires significantly less energy than conventional heating processes.

Moreover, according to a second aspect, it is the problem of the present invention to provide a dental furnace for preparing an indirect dental restoration in a process of the present invention.

Finally, according to a third aspect, it is the problem of the present invention to provide a use of the dental furnace according to the present invention for heat treating a dental restoration comprising glass ceramics.

The problem of the invention according to the first aspect is solved by a process for preparing a dental restoration comprising the following steps:
(a1) providing a dental glass ceramics mill blank and machining an indirect dental restoration from the mill blank; or
(a2) providing a dental glass ceramics press blank and pressing an indirect dental restoration from the press blank; and
(b) heat treating the indirect dental restoration obtained in step (a) or (b) at a temperature below the melting point of the dental glass ceramics for at least 15 seconds by irradiating light having a wavelength in the range of 350 to 500 nm, for preparing the dental restoration.

The problem according to the second aspect is solved by a dental furnace for preparing an indirect dental restoration, comprising:
(i) a chamber adapted to receive a dental restoration for exposure to irradiation;
(ii) one or more light sources emitting UV-light at a wavelength in the range of from 350 to 500 nm; and
(iii) means for measuring the surface temperature of the dental restoration.

The problem according to the third aspect is solved by a use of a dental furnace according to the second aspect for heat treating a dental restoration comprising glass ceramics.

The present invention is based on the recognition that a dental glass ceramics mill blank or press blank may be efficiently heated to a temperature required for preparing a dental restoration by using light of a suitable wavelength and appropriate intensity. Accordingly, the time required for a heat treatment may be significantly shortened while achieving a high level of mechanical and esthetical properties. Accordingly, the process may be carried out based on a mill blank chairside by the dental practitioner in the presence of the patient whereby a temporary restoration may be avoided and at least two visits of the patient at the dentist's office may be combined into a single appointment. Furthermore, the process may be carried out based on a dental glass ceramics press blank whereby surface microdefects may be healed by a heat treatment in a short time of less than 10 minutes while achieving and maintaining a high level of mechanical and esthetical properties, so that the heat treatment may even be carried out chairside by the dental practitioner in the presence of the patient. Moreover, the process of the present invention requires significantly less energy than conventional heating processes.

### Brief Description of the Figures

Fig. 1 shows a process flow chart diagram relating to a fully crystallized glass ceramics block processed according to the present invention.
Fig. 2 shows a process flow chart diagram relating to a partially crystallized glass ceramic block processed according to the present invention.
Fig. 3 shows a process flow chart diagram relating to a pressable glass ceramic processed according to the present invention.
Fig 4 shows a process flow chart diagram relating to glazing of a dental restoration.
Fig. 5 shows a first embodiment of a dental furnace for heating dental glass ceramics restoration to prepare an indirect dental restoration according to the present invention.
Fig. 6 shows a second embodiment of a dental furnace for heating dental glass ceramics restoration to prepare an indirect dental restoration according to the present invention.
Fig. 7 shows an embodiment of a dental furnace which is known from the prior art.

### Detailed Description of the Invention

The present invention provides a process for preparing a dental restoration, in particular an indirect dental restoration. An "indirect dental restoration" refers to a dental repair or replacement that is fabricated outside of the mouth of the patient before being placed or cemented into a patient's mouth. Indirect restorations may be used when the tooth damage is too extensive for a direct restoration to be feasible, or when a more durable, long-lasting material is required that cannot be applied directly in the oral cavity. Indirect dental restorations include crowns, bridges, inlays, onlays, abutments and veneers.

The process of the present invention comprises a step (a1) of providing a dental glass ceramics mill blank and machining an indirect dental restoration from the mill blank. Alternatively, the process comprises a step (a2) of providing a dental glass ceramics press blank and forming pressing an indirect dental restoration from the press blank.

The material of dental glass ceramics mill blank for use in the process of the present invention is not particularly limited and any commonly used dental glass ceramics mill blank or press blank for fabricating dental restorations such as crowns, veneers, abutments, bridges, inlays, and onlays, may be used. For example, the dental glass ceramics blank may be made of lithium disilicate, leucite-reinforced ceramics, zirconia reinforced lithium silicate (ZLS), feldspathic ceramics, or other monochromatic or layered glass ceramics CAD/CAM blocks.

Lithium disilicate is highly preferable for its excellent strength (approximately 360-400 MPa) and high translucency, making it ideal for aesthetic restorations, especially in the anterior (front) region of the mouth. It can be both milled and then crystallized or pressed. Lithium disilicate is suitable for single crowns, veneers, abutments, inlays, onlays, and bridges.

Leucite-reinforced ceramics have lower strength compared to lithium disilicate but offer excellent aesthetics and are easy to work with. The leucite crystals within the glass matrix enhance the material's strength to some degree (about 160 MPa) and improve its thermal expansion properties, making it a good match for fusing with porcelain. Leucite-reinforced ceramics may be used for veneers, inlays, onlays, and single-unit crowns.

Zirconia-reinforced lithium silicate (ZLS) combines the aesthetics of lithium silicate with the strength added by zirconia particles and is suitable for single crowns, inlays, onlays, and small dental bridges, offering a good balance between strength and translucency.

Feldspathic ceramics are the traditional glass-based ceramics used in dentistry with moderate strength and high translucency and superior aesthetic qualities. Feldspathic ceramics may be used for veneers and other highly aesthetic cases where strength is less of a concern than appearance.

CAD/CAM blocks such as pre-shaded and multi-layered CAD/CAM blocks, which are designed to provide a natural color gradient which eliminate the need for extensive staining or layering may also be used for speeding up the production process and allowing for same-day dental restorations.

In step (a1) of the process of the present invention, a dental restoration is machined from the dental glass ceramics mill blank. Machining a dental restoration from a dental glass ceramics mill blank involves a precise, computer-controlled process that shapes the blank into a dental restoration, such as a crown, bridge, inlay, onlay, abutment or veneer. The step is typically carried out using a CAD/CAM (Computer-Aided Design/Computer-Aided Manufacturing) system. Prior to the machining, for example by milling, a digital model of the desired dental restoration is created which may be done based on a digital scan of the patient's mouth or a dental impression, which is then converted into a digital 3D model using a scanner and CAD software. Subsequently, a dentist or dental technician uses the CAD software to design the restoration, adjusting the shape, size, and fit based on the specific requirements of the patient's dental anatomy. The designed restoration is sent from the CAD software to a CAM system, which controls the milling machine. After milling, the restoration may undergo additional processes such as manual detailing or surface treatment to refine its shape and surface texture.

The process of the present invention comprises a step (b) of heat treating the indirect dental restoration obtained in step (a1) or (a2) at a temperature below the melting point of the dental glass ceramics for at least 15 seconds by irradiating light having a wavelength in the range of 350 to 500 nm, for preparing the dental restoration.

Preferably, the dental glass ceramics mill blank comprises lithium disilicate or leucite.

Preferably, the dental glass ceramics mill blank is in an at least partially crystalline state.

Preferably, for improving the milled surface of the dental restoration, the dental restoration is heat treated to a temperature in the range of from 730 to 850 °C, more preferably 750 to 830 °C, still more preferably 760 to 820 °C. The dental restoration is heat treated for improving the milled surface of the dental restoration, preferably for at least 15 seconds to at most 3 minutes. More preferably, the dental restoration is heat treated for at least 30 seconds to at most 2 minutes.

According to a preferred embodiment, the dental glass ceramics mill blank is in a pre-crystallized state. The dental glass ceramics mill blank is in a pre-crystallized state when the material is not in a fully crystallized state. Specifically, the term pre-crystallized refers to a state of the material where the initial stages of crystallization have already occurred, but the material is not fully crystallized. This partial crystallization process is carefully controlled during the manufacturing of the glass ceramics to achieve a specific microstructure that facilitates further processing and final crystallization. In the pre-crystallized state, the material contains a mixture of amorphous (glassy) and crystalline phases. This means that the glass matrix has started to develop crystalline structures, but the transformation is not complete. A pre-crystallized state may be identified and quantified by using a method such as X-ray diffraction (XRD) or differential scanning calorimetry.

Preferably, for crystallizing the dental restoration the dental glass ceramics mill blank is heat treated at a temperature in the range of from 830 to 870 °C, more preferably in the range of from 840 to 860 °C. For crystallizing the dental restoration the dental glass ceramics mill blank is preferably heat treated for at most 6 minutes, more preferably for at most 5 minutes, still more preferably at most 4 minutes.

Preferably, the process further comprises applying a glaze to the dental restoration by spraying or painting a glaze composition and irradiating with light of a wavelength in the range of from 400 to 500 nm for a predetermined time.

In particular, the glaze preferably comprises an amount of at least 200 ppm of metal oxides selected from one or more oxides of Ce, Tb, Sr, Gd, Y, and Bi.

According to a preferred embodiment, the irradiated light has two or more intensity peaks in the range of from 350 to 500 nm. For example, the irradiated light may have one or more peaks in the range of from 350 to 400 nm and one or more peaks in the range of more than 400 to 500 nm. According to a preferred embodiment, the irradiation is continuous. Alternatively, or additionally, the irradiation may be in form of one or a plurality of consecutive light pulses. The intensity peaks of the light sources are subject to the specification of the manufacturer. Alternatively, the intensity peaks may be determined using a conventional UV spectrometer.

The irradiation may be continuous and/or in form of one or a plurality of consecutive light pulses.

Preferably, the dental glass ceramics mill blank has a three-point flexural strength in the range of from 200 to 800 MPa measured according to ISO 6872.

Preferably, the dental glass ceramics mill blank has a density in the range of from 2.0 to 3.0 g/cm³. The density may be measured by using the buoyancy method (based on the Archimedes principle) to estimate the volume. The sample mass is measured in air and in a liquid with a known density. Then the volume of the sample can be calculated based on the liquid density. Based on the mass and the volume of the sample, the density of the sample may be calculated. A standard test method for determining density is subject to DIN EN ISO 1183-1 :2019-09.

The restoration may be glazed, stained, or veneered to match the natural color of the patient's teeth and to achieve the desired aesthetic appearance. According to a preferred embodiment, the process of the present invention further comprises applying a glaze to the dental restoration by spraying or painting a glaze composition and irradiating with light of a wavelength in the range of from 400 to 500 nm for a predetermined time. Preferably, the glaze composition comprises metal oxides of Ti, Nb, Ce, Fe, Pr, and Bi, or a compound of Ti, Nb, Ce, Fe, Pr, or Bi which forms an oxide when heated in an oxidizing atmosphere. Alternatively, the glaze may be melted without additional metals.

Finally, the restoration is ready to be tested for fit and cemented into the patient's mouth.

The process according to the present invention allows to produce highly durable and aesthetically pleasing dental restorations with a precise fit, combining the advanced properties of glass ceramics and the capabilities of modern CAD/CAM technology with the convenience of a chairside treatment.

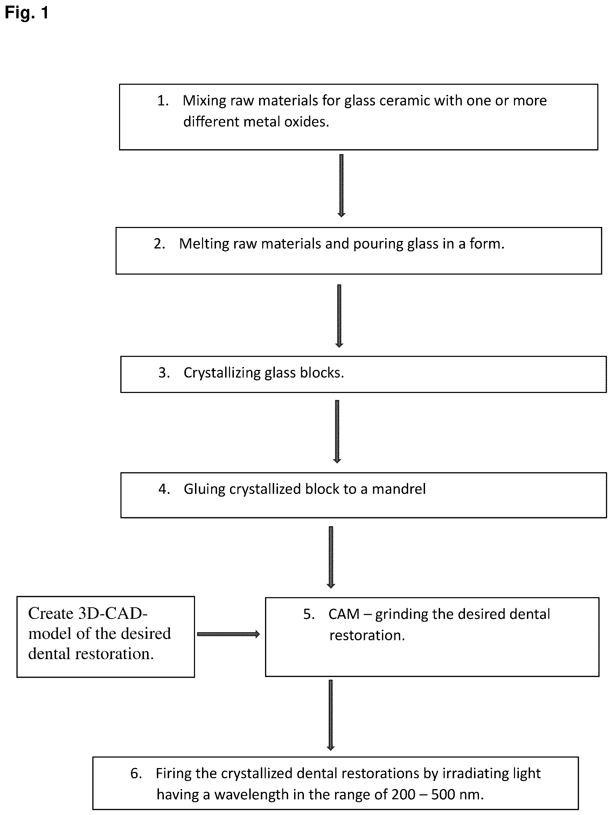

Fig. 1 shows a process flow chart diagram of an embodiment of the process of the present invention, wherein a fully crystallized glass ceramics block is used for preparing a dental restoration.

In the first step in the preparation of a dental mill block, raw materials for glass ceramic are mixed with one or more different metal oxides.

In a second step, the mixture is melted, and the glass is poured in a mold to prepare a shaped glass block. The blocks are then partially crystallized.

Subsequently, the crystallized blocks are attached to a mandrel.

Based on a 3D CAD-model of the desired dental restoration, the desired dental restoration is prepared by CAD grinding.

Finally, the crystallized dental restoration is heat-treated by irradiating light having a wavelength in the range of 350 - 500 nm for improving the surface properties of the dental restoration.

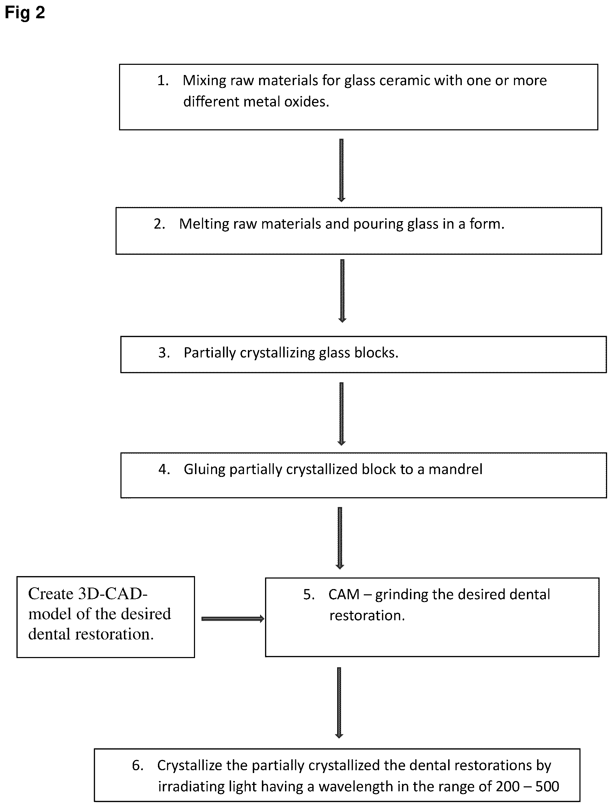

Fig. 2 shows a process flow chart diagram of a process wherein a partially crystallized glass ceramic block is used.

In a first step in the preparation of a dental mill block, raw materials for glass ceramic are mixed with one or more different metal oxides.

In a second step, the mixture is melted and the glass is poured in a mold to prepare a shaped glass ceramics block.

The blocks are then partially crystallized.

Subsequently, the partially crystallized blocks are attached to a mandrel.

Based on a 3D CAD-model of the desired dental restoration, the desired dental restoration is prepared by CAD grinding.

Finally, the partially crystallized dental restoration is heat-treated by irradiating light having a wavelength in the range of 350 - 500 nm for fully crystallizing the dental restoration.

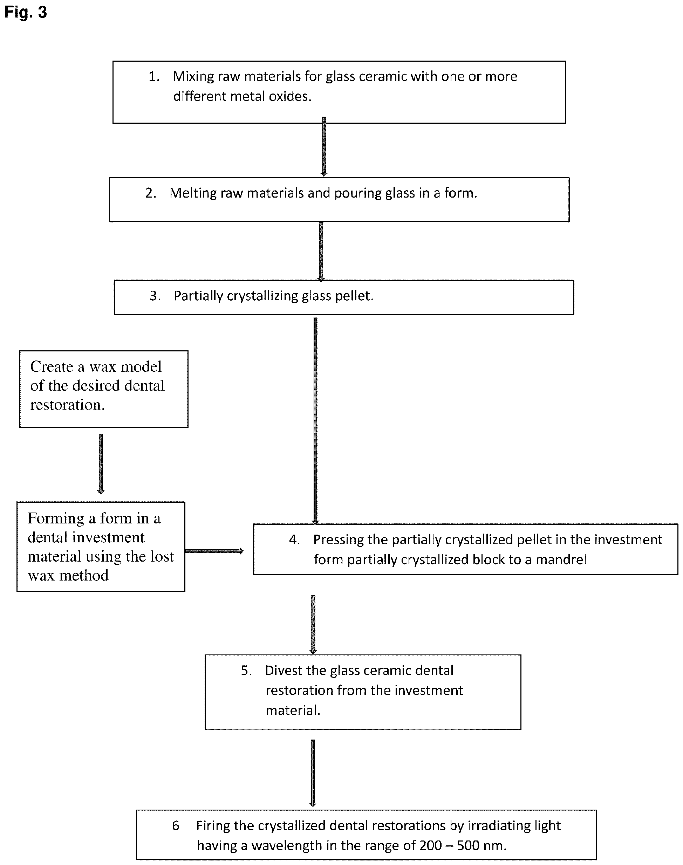

Fig. 3 shows a process flow chart diagram of a process using a pressable glass ceramic.

in a first step in the preparation of partially crystallized glass pellets, raw materials for glass ceramic are mixed with one or more different metal oxides.

In a second step, the mixture is melted, and the glass is poured in a mold to prepare a glass pellets. The pellets are then partially crystallized.

The desired dental restoration is prepared by creating a wax model of the desired dental restoration, and preparing a shape in a dental investment material using the lost wax method. The partially crystallized pellet are then pressed in the investment form. The glass ceramic dental restoration is then divested from the investment material.

Finally, the crystallized dental restoration is heat-treated by irradiating light having a wavelength in the range of 350 - 500 nm for improving the surface properties of the restoration.

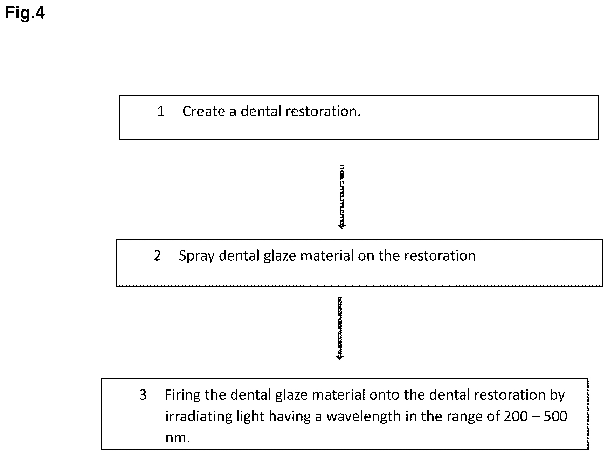

Fig. 4 shows a process flow chart diagram for glazing dental restoration. Accordingly, in a first step a dental restoration is prepared, notably according to the process of the present invention. Subsequently, a dental glaze material is sprayed on the restoration. Finally, the dental glaze material is fired onto the dental restoration by irradiating light having a wavelength in the range of 200 - 500 nm.

According to a second aspect, the present invention provides a dental furnace for heat treating glass ceramics dental restoration for preparing an indirect dental restoration

Preferably, the dental furnace is a dental chairside furnace which may be used for heat treating a glass ceramics dental restoration to prepare an indirect dental restoration in the presence of a patient. Specifically, the dental chairside furnace may be used in a single visit of the patient, which includes preparing the hard dental tissue, collecting the data for preparing a digital model of the patient's teeth, designing the indirect dental restoration, machining or pressing the indirect dental restoration, and placing the indirect dental restoration in the mouth of the patient.

The dental furnace comprises a chamber adapted to receive a dental restoration for exposure to irradiation. The chamber may be constructed from high-temperature-resistant ceramics effectively insulating the heating chamber, maintaining the required temperatures while protecting the external environment from heat. Alternatively, a stainless steel chamber with a heat-reflective coating on the interior may be provided. Advantageously, a double-walled stainless-steel chamber with an air gap or insulating material between the walls for enhanced thermal insulation may be provided whereby this design helps maintain a consistent temperature within the chamber. A chamber constructed from a high-temperature-resistant unfilled polymer material, offering excellent thermal insulation properties and durability may also be used. Composites can be designed to have low thermal conductivity, reducing heat loss during the heating process.

The dental furnace further comprises one or more light sources emitting UV-light at a wavelength in the range of from 350 to 500 nm.

UV LED arrays offering a range of wavelengths suitable for activating the photothermal effect in the glass ceramics are preferably used. Furthermore, a UV-reflective coating applied directly to the interior surfaces of the chamber is also preferred, which could be a UV-reflective paint or a thin film that covers the walls and the top, serving the same purpose as reflective panels. According to a preferred embodiment, at least two different light sources are installed in the dental chairside furnace according to the invention. Preferably, the first UV light source is working in the range of from 350 to 400 nm and the second UV light source is working in the range from above 400 to 500 nm.

According to a preferred embodiment, the one or more light sources of the dental chairside furnace emit UV-light in the range of from 350 to 500 nm at an intensity of at least 80 W/cm². Preferably, the intensity is at least 90 W/cm². Moreover, according to a preferred embodiment, a first UV light source working in the range of from 350 to 400 nm may emit UV-light at an intensity of at least 80 W/cm² and a second UV light source working in the range from more than 400 to 500 nm may emit UV-light the at an intensity of at least 60 W/cm²

The dental furnace comprises means for measuring the surface temperature of the dental restoration. Preferably, the means for measuring the temperature of a dental restoration measure the surface temperature in real-time during the heat treatment in the dental furnace for ensuring the quality and integrity of the final product.

Precise temperature control and monitoring allow for the optimization of the heating parameters, leading to restorations with superior mechanical properties and aesthetics. Several methods can be employed to measure the temperature efficiently in real-time. Preferably, pyrometers, also known as optical or radiation pyrometers, measure the temperature of an object based on the color of the visible light it emits at high temperatures. Infrared (IR) sensors or pyrometers can measure the temperature of an object without physical contact, based on the infrared radiation emitted by the object. By focusing on the dental restoration, an IR sensor can provide real-time temperature readings.

According to a preferred embodiment, the means for measuring the surface temperature of the dental restoration is a pyrometer and the dental furnace further comprises means for preventing infrared light having a wavelength in the operating range of the pyrometer, which is emitted from the light sources, from entering the pyrometer.

The dental furnace may also be equipped with advanced sensor technology embedded within the furnace chamber, designed to monitor the temperature distribution accurately. These sensors can provide real-time feedback to the furnace control system, ensuring uniform temperature and optimal heat treatment conditions. The integration of temperature measurement techniques with the furnace's control system allows for precise control over the heat treatment parameters, leading to consistent and high-quality dental restorations.

Preferably, a control device for controlling the one or more light sources, preferably in response to a set of parameters forms part of the dental furnace. According to a preferred embodiment, the dental furnace comprises a device control unit which is a microcontroller-based control unit with a touchscreen interface, offering users the ability to easily select heating programs, adjust settings, and monitor the process in real-time. An advanced programmable logic controller (PLC) with an integrated touchscreen interface is preferred. Furthermore, a computer-controlled unit integrated with dental CAD/CAM software, allowing for automated heating cycles based on the specific restoration design being produced may also be used, which may include an LCD and keypad for manual overrides and adjustments. Preferably, a digital control system with remote monitoring and control capabilities may be provided which is connected to a network, allowing operators to control the heating process and receive updates via a computer or mobile device.

Preferably, the dental furnace further comprises a control device for controlling the one or more light sources, preferably in response to a set of parameters.

Preferably, the dental furnace further comprising reflecting means for directing radiation to a maximum of the surface area of a dental restoration.

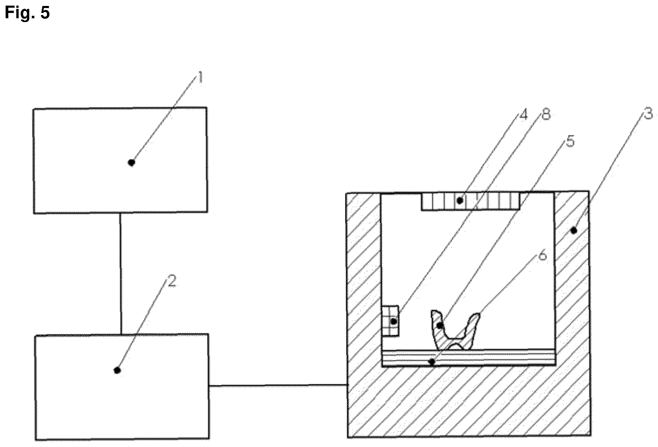

Fig. 5 shows a first embodiment of a dental furnace for heat treating a dental restoration to prepare an indirect dental restoration according to the present invention. A user interface 1 is linked to a device control unit 2 controlling a furnace comprising a housing made of a heat shielding material 3.

Preferably, the user interface 1 and the device control unit 2 may be a microcontroller-based control unit with a touchscreen interface, offering users the ability to easily select heating programs, adjust settings, and monitor the process in real-time. An advanced programmable logic controller (PLC) with an integrated touchscreen interface is preferred. Furthermore, a computer-controlled unit integrated with dental CAD/CAM software, allowing for automated heating cycles based on the specific restoration design being produced may also be used, which may include an LCD and keypad for manual overrides and adjustments. Preferably, a digital control system with remote monitoring and control capabilities may be provided which is connected to a network, allowing operators to control the heating process and receive updates via a computer or mobile device.

The housing comprises a heating chamber wherein a first UV-light source 4 is provided.

The heating chamber may be constructed from high-temperature-resistant ceramics effectively insulating the heating chamber, maintaining the required temperatures while protecting the external environment from heat. Alternatively, a stainless-steel chamber with a heat-reflective coating on the interior may be provided. Advantageously, a double-walled stainless-steel chamber with an air gap or insulating material between the walls for enhanced thermal insulation may be provided whereby this design helps maintain a consistent temperature within the heating chamber. A chamber constructed from a high-temperature-resistant composite material, offering excellent thermal insulation properties and durability may also be used. Composites can be designed to have low thermal conductivity, reducing heat loss during the heating process.

The heating chamber is adapted to receive an indirect dental restoration 5 on a heat resistant tray 6. The heat resistant tray 6 is made from a material with a high melting point and low thermal conductivity, designed to minimize heat absorption and prevent any alteration in the heating process due to the tray itself. For example, Al₂O₃ or ZrO₂ materials offer excellent strength and stability at high temperatures, with minimal thermal expansion.

The heating chamber further comprises a temperature unit for measuring the surface temperature 8 of the dental restoration.

Moreover, a light reflection material may be provided in the heating chamber. A series of adjustable mirrors made from high-reflectivity materials such as polished aluminum, or silver coatings may be used as reflecting means. These mirrors can be positioned at angles to reflect UV light to the underside or sides of the dental restoration, ensuring even exposure. Alternatively, a reflective dome or chamber surrounding the heating area, coated with a UV-reflective material may be used. This design ensures that UV light is diffused and directed from all angles toward the restoration, minimizing shadows and promoting uniform heating. Furthermore, a UV-reflective coating applied directly to the interior surfaces of the chamber is also preferred, which could be a UV-reflective paint or a thin film that covers the walls and the top, serving the same purpose as reflective panels.

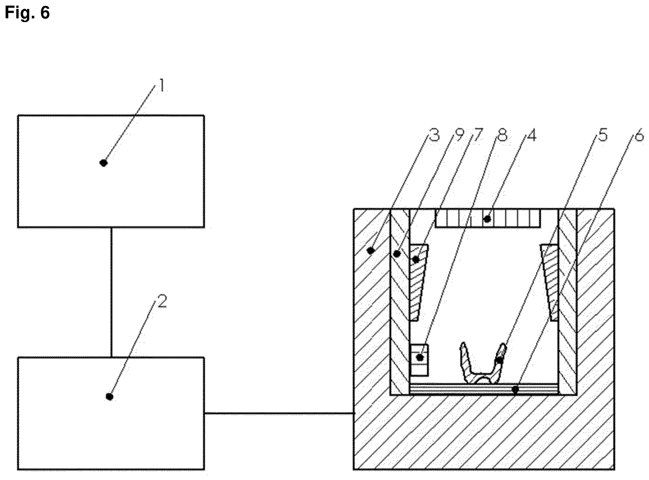

Fig. 6 shows a second embodiment of a dental furnace for heat treating a dental restoration to prepare an indirect dental restoration according to the present invention. The second embodiment is similar to the first embodiment in that a user interface 1 is linked to a device control unit 2 controlling a furnace comprising a housing made of a heat shielding material 3. The housing comprises a heating chamber wherein a first UV-light source 4 is provided at the ceiling of the heating chamber for irradiating the dental restoration from the top. Furthermore, second UV-light sources 7 are provided which are attached to the side walls of the heating chamber for providing additional irradiation. The irradiation may be of the same wave length and/or intensity as the irradiation of the first UV-light source. However, according to a preferred embodiment, the irradiation may be of a different wave length and/or intensity as the irradiation of the first UV-light source. The heating chamber comprises a temperature unit for measuring the surface temperature 8 of the dental restoration. Moreover, a light reflection material 9 is provided in the heating chamber

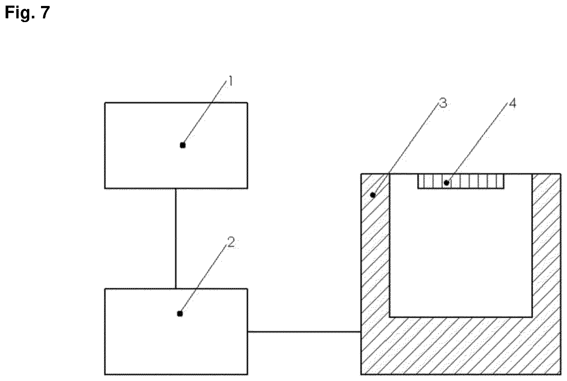

Fig. 7 shows an embodiment of a dental furnace according to the prior art for heat treating a dental restoration to prepare an indirect dental restoration. A user interface 1 is linked to a device control unit 2 controlling a furnace comprising a housing made of a heat shielding material 3.

The present invention also provides a use of a dental furnace according to the second aspect for heat treating a dental restoration comprising glass ceramics, preferably in a process according to the first aspect of the present invention.

### Reference numerals:

1. User Interface
2. Device Control Unit
3. Heat Shielding Material
4. First UV-Light Source
5. Indirect Dental Restoration
6. Heat Resistant Tray
7. Second UV-Light Source
8. Temperature Unit
9. Light Reflection Material

## Claims

1. A process for preparing a dental restoration comprising the following steps:
(a1) providing a dental glass ceramics mill blank and machining an indirect dental restoration from the mill blank, or
(a2) providing a dental glass ceramics press blank and pressing an indirect dental restoration from the press blank; and
(b) heat treating the indirect dental restoration obtained in step (a1) or (a2) at a temperature below the melting point of the dental glass ceramics for at least 15 seconds by irradiating light having a wavelength in the range of 350 to 500 nm,
for preparing the dental restoration.

2. The process according to claim 1, wherein the dental glass ceramics mill blank comprises lithium disilicate or leucite.

3. The process according to claim 1 or 2, wherein the dental glass ceramics mill blank is in an at least partially crystalline state.

4. The process according to claim 3, wherein the dental restoration is heat treated to a temperature in the range of from 730 to 850 °C for at least 15 seconds to at most 3 minutes, for improving the milled surface of the dental restoration.

5. The process according to claim 1 or 2, wherein the dental glass ceramics mill blank is in a pre-crystallized state.

6. The process according to claim 5, wherein the dental glass ceramics mill blank is heat treated at a temperature in the range of from 830 to 870 °C for at most 6 minutes, for crystallizing the dental restoration.

7. The process according to any one of the preceding claims, wherein the process further comprises applying a glaze to the dental restoration by spraying or painting a glaze composition and irradiating with light of a wavelength in the range of from 400 to 500 nm for a predetermined time.

8. The process according to claim 7, wherein the glaze comprises an amount of at least 200 ppm of metal oxides selected from one or more oxides of Ce, Tb, Sr, Gd, Y, and Bi.

9. The process according to any one of the preceding claims, wherein the irradiated light has two or more intensity peaks in the range of from 200 to 500 nm.

10. The process according to any one of the preceding claims, wherein the irradiation is continuous and/or in form of one or a plurality of consecutive light pulses.

11. The process according to any one of the preceding claims, wherein the dental glass ceramics mill blank has a three-point flexural strength in the range of from 200 to 800 MPa measured according to ISO 6872; and/or
wherein the dental glass ceramics mill blank has a density in the range of from 2.0 to 3.0 g/cm³.

12. A dental furnace for preparing an indirect dental restoration, comprising:
(i) a chamber adapted to receive a dental restoration for exposure to irradiation;
(ii) one or more light sources emitting UV-light at a wavelength in the range of from 350 to 500 nm; and
(iii) means for measuring the surface temperature of the dental restoration.

13. The dental furnace according to claim 12, wherein the means for measuring the surface temperature of the dental restoration is a pyrometer and the dental furnace further comprises means for preventing infrared light having a wavelength in the operating range of the pyrometer, which is emitted from the light sources, from entering the pyrometer.

14. The dental furnace according to claim 12 or 13, wherein at least two different light sources are installed, the first UV light source is working in the range of from 350 to 400 nm and the second UV light source is working in the range from more than 400 to 500 nm, and/or
further comprising a control device for controlling the one or more light sources, preferably in response to a set of parameters, and/or
the one or more light sources emit UV-light in the range of from 350 to 500 nm at an intensity of at least 80 W/cm²; and/or
the first UV light source working in the range of from 350 to 400 nm emits UV-light at an intensity of at least 80 W/cm² and the second UV light source working in the range from more than 400 to 500 nm emits UV-light the at an intensity of at least 60 W/cm²
dental furnace further comprising reflecting means for directing radiation to a maximum of the surface area of a dental restoration.

15. A use of a dental furnace according to any one of claims 12 to 14 for heat treating a dental restoration comprising glass ceramics.
